(19) Europäisches Patentamt
European Patent Office
Office européen des brevets

(11) Veröffentlichungsnummer: **0 051 564**
**A1**

(12) # EUROPÄISCHE PATENTANMELDUNG

(21) Anmeldenummer: **81810424.2**

(22) Anmeldetag: **26.10.81**

(51) Int. Cl.[3]: **C 07 D 249/12**
C 07 D 215/22, C 07 D 239/34
C 07 D 239/47, C 07 D 239/56
C 07 D 307/86, C 07 C 125/067
A 01 N 47/18

(30) Priorität: **31.10.80 CH 8119/80**
**02.06.81 CH 3598/81**

(43) Veröffentlichungstag der Anmeldung:
**12.05.82 Patentblatt 82/19**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Fricker, Urs**
**Baumgartenweg 8**
**CH-4460 Gelterkinden(CH)**

(72) Erfinder: **Martin, Henry, Dr.**
**Jupiterstrasse 17**
**CH-4123 Allschwil(CH)**

(72) Erfinder: **Böger, Manfred**
**Wilhelm Glock-Strasse 14**
**D-7858 Weil am Rhein 5(DE)**

(72) Erfinder: **Drabek, Jozef, Dr.**
**Benkenstrasse 12**
**Ch-4104 Oberwil(CH)**

(54) **Neue Carbamate.**

(57) N-(2-Cyanoäthyl)-N-methylcarbamate der Formel

$$NCCH_2CH_2\overset{\displaystyle CH_3}{\overset{|}{N}}COOR_1$$

worin $R_1$ Dihydrobenzofuranyl, Naphthyl, Pyrimidinyl, Pyrazolyl, Triazolyl, Chinolinyl oder Tetrahydrochinolinyl bedeutet, Verfahren zur Herstellung dieser Carbamate und ihre Verwendung in der Schädlingsbekämpfung werden beschrieben.

EP 0 051 564 A1

Croydon Printing Company Ltd.

CIBA-GEIGY AG
Basel (Schweiz)

5-13128/1+2

Neue Carbamate

Die vorliegende Erfindung betrifft N-(2-Cyanoäthyl)-N-methylcarbamate, Verfahren zu ihrer Herstellung und ihre Verwendung in der Schädlingsbekämpfung. Die N-(2-Cyanoäthyl)-N-Methylcarbamate haben die Formel

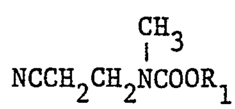

(I)

worin $R_1$ Dihydrobenzofuranyl, Naphthyl, Pyrimidinyl, Pyrazolyl, Triazolyl, Chinolinyl oder Tetrahydrochinolinyl bedeutet.

Die Gruppen bei $R_1$ können unsubstituiert oder durch ein oder mehrere $C_1$-$C_4$-Alkyl, vorzugsweise Methyl, Halogen oder Dimethylamino substituiert sein. Unter Halogen ist Fluor, Chlor, Brom oder Jod, insbesondere aber Chlor zu verstehen.

Die Verbindungen der Formel I können nach an sich bekannten Methoden z.B. wie folgt hergestellt werden:

1) 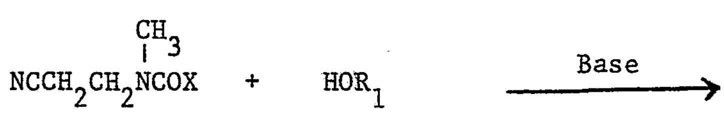

(I)

(II) (III)

2) 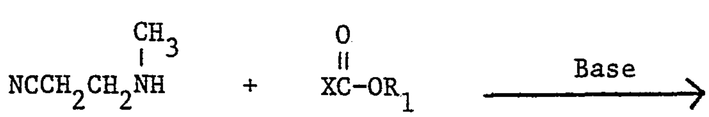

(I)

(IV) (V)

In den Formeln III und V hat $R_1$ die für die Formel I angegebene Bedeutung. In den Formeln II und V steht X für ein Halogenatom, insbesondere für ein Chloratom.

Die Verfahren werden bei einer Reaktionstemperatur zwischen -50°C und +130°C, vorzugsweise zwischen -10°C und +100°C, bei normalem oder leicht erhöhtem Druck und in Gegenwart eines gegenüber den Reaktionsteilnehmern inerten Lösungs- oder Verdünnungsmittels vorgenommen.

Als geeignete Basen für die Verfahren kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z.B. Kalium-tert.butylat und Natriummethylat in Betracht.

Als Lösungs- oder Verdünnungsmittel eignen sich z.B. Aether und ätherartige Verbindungen wie Diäthyläther, Di-iso-propyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangsstoffe der Formeln II bis V sind bekannt bzw. können analog bekannten Methoden hergestellt werden.

Die Verbindungen der Formel I eignen sich zur Bekämpfung von Schädlingen an Tieren und Pflanzen. So haben diese Verbindungen fungizide und pflanzenregulatorische Eigenschaften.

Insbesondere eignen sich die Verbindungen der Formel I zur Bekämpfung von Insekten z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Acarina.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

In diesem Zusammenhang ist hervorzuheben, dass die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z.B. Musca domestica und Mückenlarven. Daneben zeichnen sich die Verbindungen der Formel I durch eine breite ovizide und ovilarvizide Wirkung aus.

Darüber hinaus besitzen die Verbindungen der Formel I eine wertvolle Wirkung gegen pflanzenparasitäre Nematoden sowie gegen ektoparasitäre Milben und Zecken z.B. der Familien Ixodidae, Argasidae und Dermanyssidae.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den

angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien oder zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seiten wie wasserlösliche synthetische oberflächenaktive Verbindungen sein.

Als Seiten eignen sich die Alkali-, Erdalkali- oder gegebenenfalls substituierten Ammoniumsalze von höheren Fettsäuren ($C_{10}$-$C_{22}$), wie z.B. die Na- oder K-Salze oder Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alky-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Formulierungsbeispiele für flüssige Wirkstoffe der Formel I</u>

<u>(% = Gewichtsprozent)</u>

| 1. <u>Emulsions-Konzentrate</u> | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | - |

| | | |
|---|---|---|
| Na-Diisobutylnaphthalinsulfonat | - | 6% |
| Octylphenolpolyäthylglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

Beispiel 1: Herstellung von N-(2-Cyanoäthyl)-N-methyl-[2-isopropyl-4-methyl-pyrimidinyl(6)]-carbamat.

3,04 g 2-Isopropyl-4-methyl-6-hydroxy-pyrimidin, 80 ml Methyläthylketon und 3,31 g $K_2CO_3$ werden eine Stunde unter Rückfluss gerührt. Nach der Abkühlung auf 20°C werden 2,95 g N-(2-Cyanoäthyl)-N-methylcarbamoylchlorid gelöst in 15 ml Methyläthylketon zugetropft. Nach 20-stündigem Rühren bei 50°C wird das Reaktionsgemisch abgenutscht. Nach dem Abdestillieren des Lösungsmittels aus dem Filtrat wird das Rohprodukt aus einem Gemisch von Toluol/Hexan (1:1) umkristallisiert. Man erhält die Verbindung der Formel

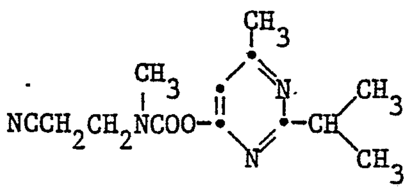

mit einem Schmelzpunkt von 77-79°C.

Auf analoge Weise werden auch folgende Verbindungen hergestellt:

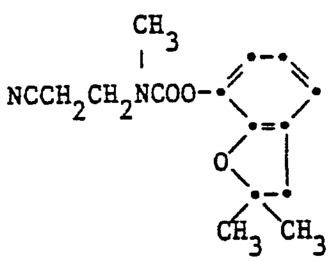

$n_D^{20°}$ = 1,5250

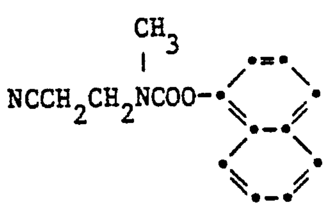

$n_D^{20°}$ = 1,5800

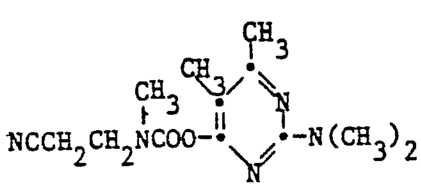

Smp.: 98-100°C

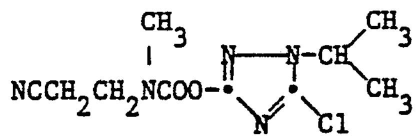

$n_D^{20°}$ = 1,4954

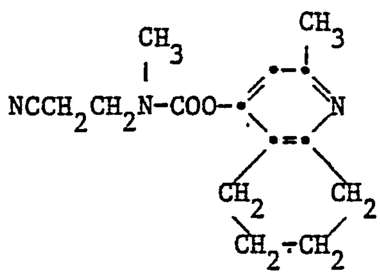

$n_D^{20°}$ = 1,5302

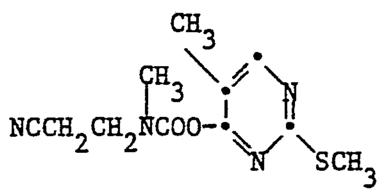

$n_D^{20°}$ = 1.5513

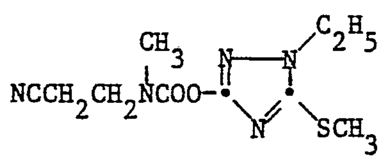

$n_D^{20°}$ = 1.5180

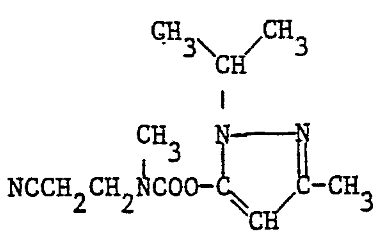

Smp.: 69-70°C

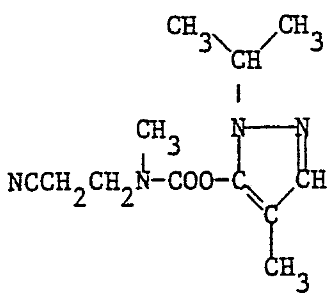

$n_D^{20°}$ = 1,4800

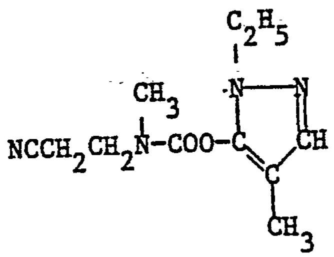

$n_D^{20°}$ = 1,5180

Beispiel 2: Insektizide Kontakt-Wirkung: Myzus persicae

In Wasser angezogene Pflanzen (Vicia fabae) wurden vor dem Versuchsbeginn je mit ca. 200 Individuen der Spezies Myzus persicae besiedelt. Die so behandelten Pflanzen wurden 3 Tage später mit einer Lösung enthaltend 10 oder 1 ppm der zu prüfenden Verbindung aus dem 30 cm Distanz bis zur Tropfnässe besprüht. Man verwendete pro Test-Verbindung und pro Konzentration zwei Pflanzen, und eine Auswertung der erzielten Abtötungsrate erfolgte nach weiteren 24 Stunden.

Verbindungen gemäss Beispiel 1 zeigten die in der folgenden Tabelle angegebene Wirkung gegen Insekten der Spezies Myzus persicae.

Beispiel 3: Insektizide systemische Wirkung: Aphis craccivora

Bewurzelte Bohnenpflanzen wurden in Töpfe, welche 600 ccm Erde enthalten, verpflanzt. Anschliessend wurden 50 ml einer Versuchslösung enthaltend 25 ppm, 5 ppm bzw. 1 ppm der zu prüfenden Verbindung direkt auf die Erde gegossen.

Nach 24 Stunden wurden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgte 24 und 48 Stunden nach Versuchsbeginn. Pro Konzentrationsdosis Testsubstanz wurden zwei Pflanzen, je eine in einem separaten Topf, verwendet. Der Versuch wurde bei 25°C und 70% relativer Luftfeuchtigkeit durchgeführt.

Verbindungen gemäss Beispiel 1 hatten die in der folgenden Tabelle angegebene Wirkung gegen Insekten der Spezies Aphis craccivora.

Biologische Versuchsergebnisse

In der folgenden Tabelle sind Versuchsergebnisse auf der Basis der vorstehenden Beispiele aufgeführt, und zwar mit folgendem Bewertungsindex in Bezug auf die prozentuale Abtötung der Schädlinge:

A: 70-100% Abtötung bei 1 ppm Wirkstoffkonzentration
B: 70-100% Abtötung bei 5 ppm Wirkstoffkonzentration
C: 70-100% Abtötung bei 10 ppm Wirkstoffkonzentration
D: 70-100% Abtötung bei 25 ppm Wirkstoffkonzentration

| Verbindungen<br>$CH_3$<br>$NC-CH_2CH_2NCOOR'$<br>R | Wirksamkeit gegen<br>Mycus persicae | <br>Aphis craccivora |
|---|---|---|
| 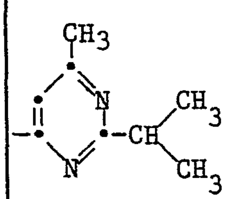 | A | A |
| 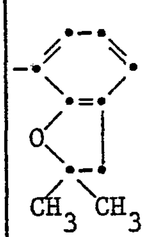 | A | A |
| 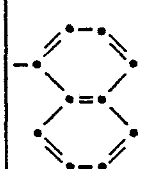 | C | D |

| Verbindungen 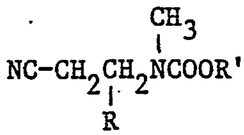 | Wirksamkeit gegen Mycus persicae | Aphis craccivora |
|---|---|---|
| 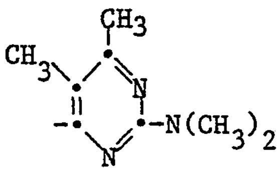 | A | A |
| 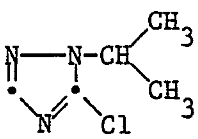 | A | B |
| 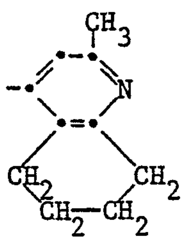 | C | D |
| 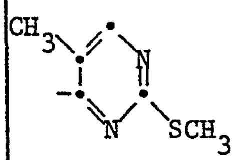 | C | D |
| 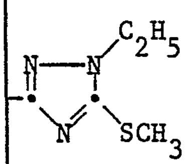 | C | D |
| 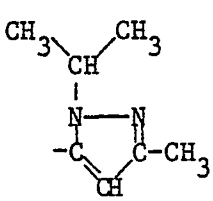 | C | D |

0051564



| Verbindungen 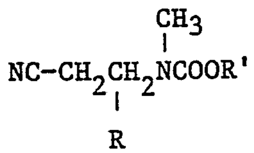 | Wirksamkeit gegen | |
|---|---|---|
| | Mycus persicae | Aphis craccivora |
| 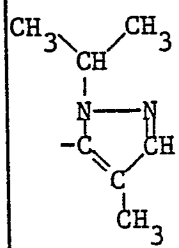 | C | A |
| 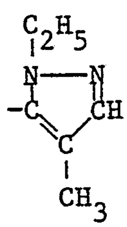 | C | D |

## Patentansprüche

1. Eine Verbindung der Formel

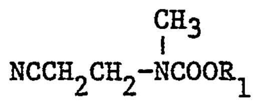

worin $R_1$ Dihydrobenzofuranyl, Naphthyl, Pyrimidinyl, Pyrazolyl, Triazolyl, Chinolinyl oder Tetrahydrochinolinyl bedeutet.

2. Die Veribndung gemäss Anspruch 1 der Formel

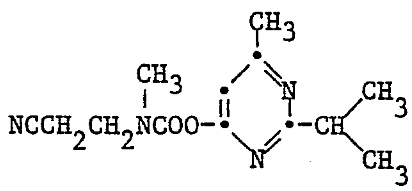

.

3. Die Verbindung gemäss Anspruch 1 der Formel

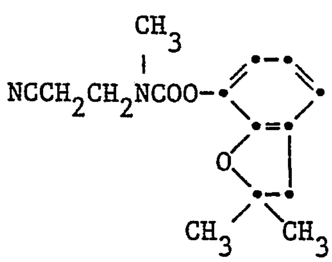

.

4. Die Verbindung gemäss Anspruch 1 der Formel

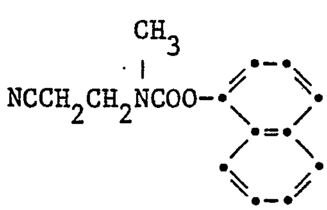

.

0051564



5. Die Verbindung gemäss Anspruch 1 der Formel

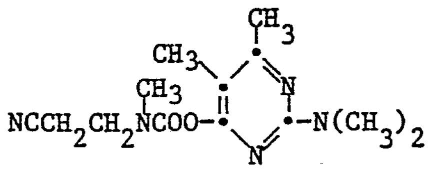 .

6. Die Verbindung gemäss Anspruch 1 der Formel

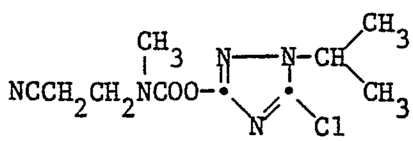 .

7. Eine Verbindung gemäss Anspruch 1 der Formel

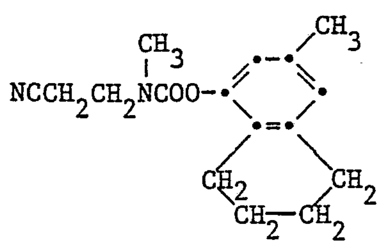 .

8. Eine Verbindung gemäss Anspruch 1 der Formel

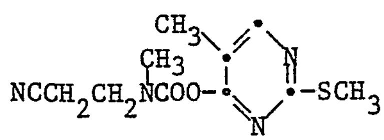 .

9. Eine Verbindung gemäss Anspruch 1 der Formel

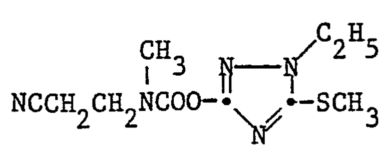 .

10. Ein Verfahren zur Herstellung von Verbindungen gemäss Anspruch 1, dadurch gekennzeichnet, dass man in Gegenwart einer Base eine Verbindung der Formel

$$NCCH_2CH_2\overset{\overset{CH_3}{|}}{N}COX \quad ,$$

worin X Halogen bedeutet, mit einer Verbindung der Formel

$$HOR_1$$

umsetzt, worin $R_1$ die im Anspruch 1 angegebene Bedeutung hat.

11. Ein Schädlingsbekämpfungsmittel, welches als aktive Komponente eine Verbindung gemäss Anspruch 1 enthält.

12. Die Verwendung einer Verbindung gemäss Anspruch 1 zur Bekämpfung von Schädlingen an Tieren und Pflanzen.

13. Die Verwendung gemäss Anspruch 12 zur Bekämpfung von Insekten und Vertretern der Ordnung Akarina.

Europäisches Patentamt

# EUROPÄISCHER RECHERCHENBERICHT

Nummer der Anmeldung

EP 81 81 0424.2

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | DE - A1 - 2 843 691 (SCHERING AG)<br>* Ansprüche 109,110 *<br>-- | 10,11 | C 07 D 249/12<br>C 07 D 215/22<br>C 07 D 239/34<br>C 07 D 239/47<br>C 07 D 239/56<br>C 07 D 307/86<br>C 07 C 125/067<br>A 01 N 47/18 |
| | DE - A1 - 2 844 811 (SCHERING AG)<br>* Ansprüche 47,48 *<br>-- | 10,11 | |
| | US - A - 3 803 208 (K. SZABO et al.)<br>* Tabelle 4 *<br>---- | 11 | |

RECHERCHIERTE SACHGEBIETE (Int. Cl.3)

A 01 N 47/18
C 07 C 125/067
C 07 D 215/22
C 07 D 239/34
C 07 D 239/47
C 07 D 239/56
C 07 D 249/12
C 07 D 307/86

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 19-01-1982 | FROELICH |